# EUROPÄISCHE PATENTSCHRIFT

(11) **EP 0 282 844 B1**
(45) Veröffentlichungstag und Bekanntmachung des Hinweises auf die Patenterteilung: **20.07.1994**
(21) Anmeldenummer: 88103417.7
(22) Anmeldetag: 05.03.1988
(51) Int. Cl.: A61K 31/13, A61K 31/48

(54) **Verwendung von Dopamin-Agonisten zur Herstellung eines Arzneimittels für die Behandlung von Knochenverletzungen**
Use of dopamine-agonists in the preparation of a medicament for the treatment of bone injuries
Utilisation des agonistes de la dopamine pour la fabrication d'un médicament pour le traitement des blessures d'os

(30) Priorität: 09.03.1987 YU 389/87
(43) Veröffentlichungstag der Anmeldung: 21.09.1988
(73) Patentinhaber: Sikiric, Predrag Dr.Sc., 41000 Zagreb (YU); Kalogjera, Livije, Zagreb (YU); Kusec, Rajko, Zagreb (YU)
(72) Erfinder: Sikiric, Predrag Dr.Sc., 41000 Zagreb (YU); Kalogjera, Livije, Zagreb (YU); Kusec, Rajko, Zagreb (YU)
(74) Vertreter: Gleiss, Alf-Olav, Dipl.-Ing.

(56) Entgegenhaltungen:
- AKTUEL. GERONTOL., Band 13, Nr. 4, 1983, Seiten 142-145, Georg Thieme Verlag, Stuttgart, DE, H. DITTMER et al;
- ANNALS OF INTERNAL MEDICINE, Band 105, Nr. 4, 1986, Seiten 543-545 American College of Physicians, US, J.A. JACKSON et al;
- MINERVA MEDICA, Band 71, 1980, Seiten 391-400, A. MUSSA et al;
- BLOOD, Band 69, Nr. 1, Januar 1987, Seiten 345-348, S. SIENA et al;
- JOURNAL OF THE AMERICAN ACADEMY OF DERMATOLOGY, Band 16, Nr. 2, Teil 1, Februar 1987, Seiten 388-389, G. WEBER et al.;
- J. RHEUMATOL., Band 11, Nr. 6, 1984, Seite 865, M.M. CERINIC

## Beschreibung

Die Erfindung betrifft die Verwendung von Dopamin-Agonisten zur Herstellung eines Arzneimittels für die Behandlung von Knochenbrüchen, von chirurgischen Eingriffen in das Skelett und/oder für die Behandlung von Pseudarthrose.

Es ist bekannt, Dopamin-Agonisten in der Medizin in verschiedenen Bereichen einzusetzen. Beispielsweise wird Bromocriptin zur Behandlung von zum Beispiel durch Hypophysentumoren hervorgerufener Hyperprolaktinämie eingesetzt. Auch die Behandlung von Galaktorrhoe, Amenorrhoe und Sterilität wurden bromocriptinhaltige Tabletten verwendet. Auch die Parkinson'sche Krankheit und Akromegalie werden mit Bromocriptin behandelt.

Ein weiterer Dopamin-Agonist, Amantadin, wurde ebenfalls schon bei der Parkinson'schen Krankheit verabreicht. Darüber hinaus wurde vorgeschlagen, bei Frakturen am proximalen Famur Dopamin einzusetzen, um die Nachteile zu mindern, die durch die Verwendung von Knochenzement bei chirurgischen Eingriffen in das Skelett auftreten (H. Dittmer et al.), "Frakturen am proximalen Famur bei über 70-jährigen-Verfahrenswahl und Ergebnisse", Aktuel. Gerontol. Band 13, Nr. 4, 1983, S. 142 bis 145). Der gezielte Einsatz von Dopamin-Agonisten zur Förderung des Heilungsprozesses des Knochens nach Verletzungen beziehungsweise chirurgischen Eingriffen oder bei Pseudarthrose finden sich hier nicht.

Es ist außerdem bekannt, Bromocriptin zur Behandlung endokriner Tumore zu verwenden, die Osteoporose nach sich ziehen (J.A. Jackson et al., "Symptomatic Osteopososis in a Man with Hyperprolactinemic Hypogonadism", Annal of internal Medicine, Band 105, Nr. 4, 1986, S. 543-545). Durch die Behandlung von Tumoren wurde jedoch die Verwendung Bromocriptin zur Förderung der Knochenheilung nicht nahegelegt.

Schließlich wurde noch vorgeschlagen, eine besondere Form der Arthritis, nämlich die Arthritis psoriatica mit Hilfe von Bromocriptin zu behandeln (G. Weber et al., "Treatment of psoriatic arhtritis with bromocriptine", Journal of the American Academy of Dermatology, Band 16, Nr. 2, Teil 1, Februar 1987, S. 388-389). Die Behandlung von Knochenbrüchen und dergleichen wird hierdurch nicht nahegelegt.

Es hat sich nun herausgestellt, daß Dopamin-Agonisten bei der Herstellung von Arzneimitteln für die Behandlung von Knochenverletzungen herangezogen werden können. Bislang waren auf diesem medizinischen Gebiet ausschließlich chirurgische Behandlungsverfahren bekannt. Der Stand der Technik ergibt keinerlei Hinweise darauf, daß Dopamin-Agonisten auf diesem Gebiet der Medizin einsetzbar sind.

Vorzugsweise können Dopamin-Agonisten in folgenden Bereichen der Behandlung von Knochenverletzungen eingesetzt werden:
1. Bei Knochenbrüchen;
2. bei operativen Eingriffen in das Skelett, bei spielsweise bei der Osteotomie;
3. bei Knochenverpflanzungen bzw. bei immunologischen Reaktionen eines Knochentransplantates gegen den Empfänger (Graft-versus-host-reaction);
4, bei Pseudarthrose, also bei der Bildung falscher Gelenke im Anschluß an Knochenbrüche, und
5. bei der Arthrodese, bei der operativen Gelenkversteifung zur Beseitigung eines zerstörten Gelenks.

Bei der Behandlung derartiger Knochenverletzungen fanden bislang Dopamin-Agonisten keine Verwendung. Anhand von Versuchen hat sich herausgestellt, daß insbesondere die Verwendung von Amantadin und Bromocriptin bei der Herstellung von Arzneimitteln für die Behandlung von Knochenverletzungen besonders geeignet ist. Die Mittel können sowohl intravenös als auch, bei der Bindung an übliche, geeignete Träger, oral verabreicht werden.

Die Wirkung der Dopamin-Agonisten wird im folgenden anhand eines Tierversuchs erläutert. Dabei wurde die Wirkung von Dopamin-Agonisten auf Knochenbrüche bei Ratten untersucht.

Bei den Versuchen wurden Albinoratten der Sorte Wister männlichen Geschlechts mit einem Gewicht von 200 g verwandt.

Eine Stunde bevor ein Knochenbruch bei den Tieren herbeigeführt wurde, wurden Amantadin in der Dosis von 20,0 g pro kg Körpergewicht intraperitoneal, bzw. Bromokrptin in einer Dosierung von 10,0 mg pro kg Körpergewicht ebenfalls intraperitoneal verabreichte. Nach Verursachung des Knochenbruchs wurde diese Dosis für die nächsten 12 Tage aufrechterhalten.

Eine Kontrollgruppe von Ratten erhielt gleichzeitig auf die gleiche Art im gleichen Umfang 0,9%ige Kochsalzlösung.

Aus den Gruppen wurden am 5., 8. und 12. Tag nach dem Trauma Tiere untersucht. In jeder untersuchten Gruppe waren 10 Ratten.

Bei dem Versuch wurde der Metarsusknochen durch digitalen Druck auf die Diaphyse hervorgerufen. Dabei wurde der rechte hintere Fuß der Ratten ausgewählt. Der Eingriff wurde unter kurzer Äther-Anästhesie durchgeführt. Eine Immobilisierung der Tiere wurde nicht durchgeführt.

Bei der anschließenden klinischen Untersuchung wurde der Stand der gebrochenen Knochen verfolgt. Es stellte sich heraus, daß die normale Entwicklung des posttraumatischen Oedems in den Gruppen, die mit Amantadin und Bromocriptin behandelt wurden, milder verlief als bei den Tieren der Kontrollgruppen. Es zeigte sich auch, daß die behandelten Tiere das verletzte Bein freier und schneller gebrauchten.

Bei den am 5, 8 und 12 Tag herausgegriffenen Tieren wurden die Pfoten pathohistologisch untersucht. Die sezierten Pfoten wurden mit histologischen Standardmethoden behandelt. Die Knochen wurden von weichem Gewebe gereinigt und dekalziniert; anschließend in Paraflast bzw Parafin eingebettet und serienmäßig geschnitten. Die Längsschnitte, die entlang dem größtem Durchmesser der Diaphyse verliefen, wurden auf Glas fixiert und in der üblichen Technik gefärbt.

Die qualitative Analyse der nach fünf Tagen entnommenen Proben hat gezeigt, daß sich die Art des Gewebes bei beiden Gruppen nicht wesentlich unterschied. Es hatte sich jeweils fibröser Kallus gebildet.

Bei den Ratten, die am 8 Tag nach dem Trauma untersucht wurden, zeigten sich Unterschiede in Art und Menge des Gewebes im Bereich des Bruchs. Während bei den mit Dopamin-Agonisten behandelten Ratten die Knochen deutlich ausgeprägt knorpeligen Kallus zeigten, war diese Art Gewebe bei der nicht behandelten Kontrollgruppe erst wenig ausgebildet.

Die deutlichsten Unterschiede zeigten sich bei den am 12 Tag untersuchten Ratten. In der Gruppe die mit Amantadin behandelt wurde, sowie in der, die Bromokriptin verabreicht bekam, war ein gut entwickelter, knöcherner Kallus mit dichter trebekulärer Struktur vorhanden, bei dem sich auch eine deutliche Transformation abzeichnete. Bei der Kontrollgruppe, die lediglich physiologische Kochsalzlösung erhalten hatte, ließ sich nur ein gut entwickelter knorpeliger Kallus nachweisen, bei dem Transformation, Resorption und Austausch mit Elementen der Osteoiden und Zellpopulationen, welche den Knochen bilden, den Osteoplasten, erst langsam anfing.

Aus den Untersuchungen zeigt Sich, daß die beschleunigte Knochenheilung bzw. Knochenbildung durch auf Dopamin beruhenden Mechanismen vermittelt wird. Dies wird darauf zurückgeführt, daß Amantadin die Absetzung von Dopamin aus präsynaptischen Bläschen erhöht und daß Bromocriptin als Agonist der Dopaminrezeptoren wirkt.

Nach allem wird daraus der Schluß gezogen, daß es möglich ist, mit Dopamin-Agonisten den Therapieverlauf bei folgenden Knochenverletzungen günstig zu beeinflussen, nämlich bei einem Trauma von Skelettknochen, bei therapeutischen Eingriffen in das Skelett, bei Knochenverletzungen, bei Pseudoarthrose sowie bei Arthrodese.

Bei der Herstellung eines Arzneimittels für die Behandlung von Knochenverletzungen können Dopamin-Agonisten sowohl als Infusionslösung als auch in Form von Tabletten bzw. Dragees verwendet werden. Es ist auch möglich, Amantadin und Bromocriptin bei der Herstellung von Arzneimitteln gemeinsam zu verwenden.

## Patentansprüche

1. Verwendung von Dopamin-Agonisten zur Herstellung eines Arzneimittels für die Behandlung von Knochenbrüchen, für die Behandlung von chirurgischen Eingriffen in das Skelett und/oder für die Behandlung von Pseudarthrose.

2. Verwendung nach Anspruch 1, **dadurch gekennzeichnet**, daß das Arnzeimittel für die Behandlung von therapeutischen Eingriffen in das Skelett herangezogen wird.

3. Verwendung nach Anspruch 1, **dadurch gekennzeichnet**, daß das Arnzeimittel für die Behandlung von transplantierten Knochenteilen herangezogen wird.

4. Verwendung nach Anspruch 1, **dadurch gekennzeichnet**, daß das Arnzeimittel für die Behandlung von durch Arthrodese versteiften Gelenken herangezogen wird.

5. Verwendung von Amantadin zur herstellung eines Arzneimittels für die Behandlung von Knochenverletzungen nach einem der Ansprüche 1 bis 4.

6. Verwendung von Bromocriptin zur Herstellung eines Arzneimittels für die Behandlung von Kochenverletzungen nach einem der Ansprüche 1 bis 4.

7. Verwendung von Amantadin und/oder Bromokriptin zur Herstellung einer Infusionslösung zur Behandlung von Knochenverletzungen nach einem der Ansprüche 1 bis 6.

8. Verwendung von Amandatin und/oder Bromokriptin zur Herstellung von Tabletten und/oder Dragees zur Behandlung von Knochenverletzungen nach einem der Ansprüche 1 bis 4.

## Claims

1. Use of dopamine agonists for preparing a medicament for the treatment of bone fractures, for the treatment of surgical interventions in the skeleton and/or for the treatment of pseudarthrosis.

2. Use according to claim 1, characterised in that the medicament is employed for the treatment of therapeutic interventions in the skeleton.

3. Use according to claim 1, characterised in that the medicament is employed for the treatment of transplanted parts of bone.

4. Use according to claim 1, characterised in that the medicament is employed for the treatment of joints stiffened by arthrodesis.

5. Use of amantadine for preparing a medicament for the treatment of injuries to the bone, according to one of claims 1 to 4.

6. Use of bromocriptin for preparing a medicament for the treatment of injuries to the bone, according to one claims 1 to 4.

7. Use of amantadine and/or bromocriptin for preparing an infusion solution for treating injuries to the bone, according to one of claims 1 to 6.

8. Use of amantadine and/or bromocriptin for preparing tablets and/or coated tablets for treating injuries to the bone, according to one of claims 1 to 4.

## Revendications

1. Utilisation d'antagonistes de la dopamine à la préparation d'un médicament pour le traitement de fractures osseuses, pour le traitement d'interventions chirurgicales dans le squelette et/ou pour le traitement de la pseudoarthrose.

2. Utilisation selon la revendication 1, caractérisée en ce que le médicament est utilisé pour le traitement d'interventions thérapeutiques dans le squelette.

3. Utilisation selon la revendication 1, caractérisée en ce que le médicament est utilisé pour le traitement de parties osseuses transplantées.

4. Utilisation selon la revendication 1, caractérisée en ce que le médicament est utilisé pour le traitement d'articulations raidies par arthrodèse.

5. Utilisation de l'amantadine à la préparation d'un médicament pour le traitement de lésions osseuses selon une des revendications 1 à 4.

6. Utilisation de la bromocriptine à la préparation d'un médicament pour le traitement de lésions osseuses selon une des revendications 1 à 4.

7. Utilisation de l'amantadine et/ou de la bromocriptine à la préparation d'une solution d'infusion pour le traitement de lésions osseuses selon une des revendications 1 à 6.

8. Utilisation de l'amantadine et/ou de la bromocriptine à la préparation de comprimés et/ou de pilules pour le traitement de lésions osseuses selon une des revendications 1 à 4.
